# EUROPEAN PATENT APPLICATION

(11) **EP 2 018 908 A1**
(43) Date of publication of application: **28.01.2009**
(21) Application number: 08160332.6
(22) Date of filing: 14.07.2008
(51) Int. Cl.: B01L 3/02, A61B 17/34, B41J 2/01, C12M 3/00, C12N 15/87

(54) **Discharge apparatus**

(30) Priority: 18.07.2007 JP 2007187679
(71) Applicant: Fujitsu Limited, Kawasaki-shi, Kanagawa 211-8588 (JP)
(72) Inventor: Nishio, Chikara c/o FUJITSU LIMITED, Kawasaki-shi, Kanagawa 211-8588 (JP); Nishiyama, Shusaku c/o FUJITSU LIMITED, Kawasaki-shi, Kanagawa 211-8588 (JP)
(74) Representative: Stebbing, Timothy Charles

(57) **Abstract**

According to an embodiment of the invention, a discharge apparatus for discharging a liquid includes a discharge outlet (26) for discharging the liquid; a first conduit (28) extending toward the discharge outlet; and a second conduit (39) having one end connected to the first conduit for flowing a gas therethrough the second conduit extending generally in the direction toward the discharge outlet and generally approaching the first conduit towards the portion where the second conduit is connected to the first conduit. The discharge apparatus further includes a chamber (35) connected to the other end of the second conduit; and a heater (36) installed in the chamber for heating a gas in the chamber so as to cause the heated gas to flow into the second conduit and to push out the liquid out of the discharge outlet.

## Description

This art relates to a discharge apparatus, for example. Such a discharge apparatus and an injection apparatus are discussed in, for example, Japanese Laid-open Patent Publications No. 04-289457, No. 2006-166756, No. 2002-286732, and No. 2004-337734.

For example, a microinjection capillary is discussed in Japanese Laid-open Patent Publications No. 2006-166756. A first liquid layer to be injected to cells is held at the tip of the capillary. A second liquid layer is held on the first liquid layer in the capillary. The first and second liquid layers define an interface therebetween. A laser light absorber is dispersed into the second liquid layer, for example. The laser absorber absorbs laser light and generates light. As a result, the second liquid layer thermally expands. An expansion pressure of the second liquid layer is converted to an injection pressure of the first liquid layer, with the result that the first liquid layer is injected from the tip of the capillary. In this way, a liquid is injected to cells, for example.

Such a capillary has a problem of the laser light absorber in the second liquid layer mixing with the first liquid layer through the interface therebetween. The laser light absorber mixed with the first liquid layer is injected to cells. In addition, the first and second liquid layers should be layered in the capillary. It takes a lot of time and effort to form such a laminate. As a result, microinjection costs are high.

It is desirable to provide a discharge apparatus, by which improved controllability of liquid discharge is achieved.

According to an aspect of an embodiment, a discharge apparatus for discharging a liquid includes a discharge outlet for discharging the liquid; a first conduit extending toward the discharge outlet; a second conduit having one end connected to the first conduit for flowing a gas therethrough the second conduit extending generally in the direction toward the discharge outlet and generally approaching the first conduit toward the portion where the second conduit is connected to the first conduit; a chamber connected to the other end of the second conduit; and a heater installed to the chamber for heating a gas in the chamber so as to cause the heated gas to flow into the second conduit and to push out the liquid out of the discharge outlet.

Hereinafter, an embodiment of the present invention will now be described, by way of example only, with reference to the accompanying drawings, in which:
FIG. 1 schematically shows the structure of a liquid injection apparatus according to an embodiment;
FIG. 2 is a sectional view schematically showing the structure of a liquid injection apparatus according to a first example;
FIG. 3 is a block diagram of a control system;
FIG. 4 schematically shows how to inject medical agent to cells;
FIG. 5 is a flowchart of medical agent injection flow;
FIG. 6 schematically shows how to inject medical agent to cells;
FIG. 7 schematically shows how to inject medical agent to cells;
FIG. 8 is a sectional view schematically showing the structure of a liquid injection apparatus according to a second example;
FIGs. 9A to 9C are partial perspective views schematically showing the structure of a capillary; and
FIGs. 10A to 10C are partial perspective views schematically showing the structure of a capillary.

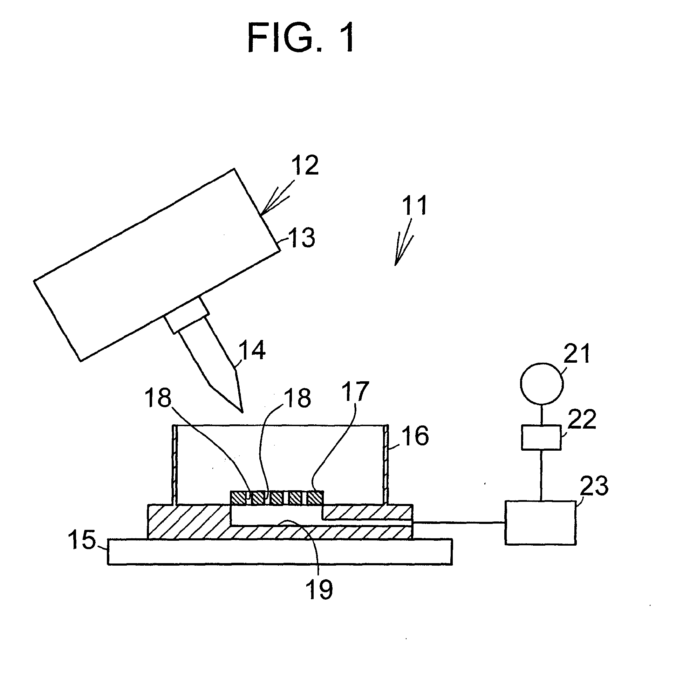

FIG. 1 schematically shows the structure of a microinjection apparatus 11 according to an embodiment. The microinjection apparatus 11 incorporates an infusion unit 12 for injecting medical agent or DNA into a minute (or small) object such as a cell. The infusion unit 12 includes a liquid discharge apparatus 13. The liquid discharge apparatus 13 is described in detail below. The liquid discharge apparatus 13 is provided with a capillary 14. The capillary 14 holds a liquid to be injected into a cell. For example, the capillary 14 is made of glass. The infusion unit 12 can move to and fro along the axis of the capillary 14. A supporting unit 24 is provided opposite to an operation stage 15. The infusion unit 12, that is, the capillary 14 can move relative to the operation stage 15.

A dish 16 is provided on the level surface of the operation stage 15. A culture solution is poured into the dish 16. Cells to be injected with an agent are dispersed in the culture solution. A silicon chip 17 is secured into the inside of the dish 16. The silicon chip 17 has a plurality of through-holes 18 that pass through the chip from the front side to the rear side. A path 19 formed in the dish 16 is connected to each through-hole 18. A vacuum pump 21 is connected to the path 19. The vacuum pump 21 generates a negative pressure. With the generation of a negative pressure, air or liquid can be sucked out of the path 19. A pressure regulating valve 22 is connected to the vacuum pump 21. The pressure regulating valve 22 adjusts the negative pressure applied to the path 19 by the vacuum pump 21. A tank 23 is provided between the path 19 and the vacuum pump 21. As described below, the tank 23 stores a culture solution that flows from the dish 16.

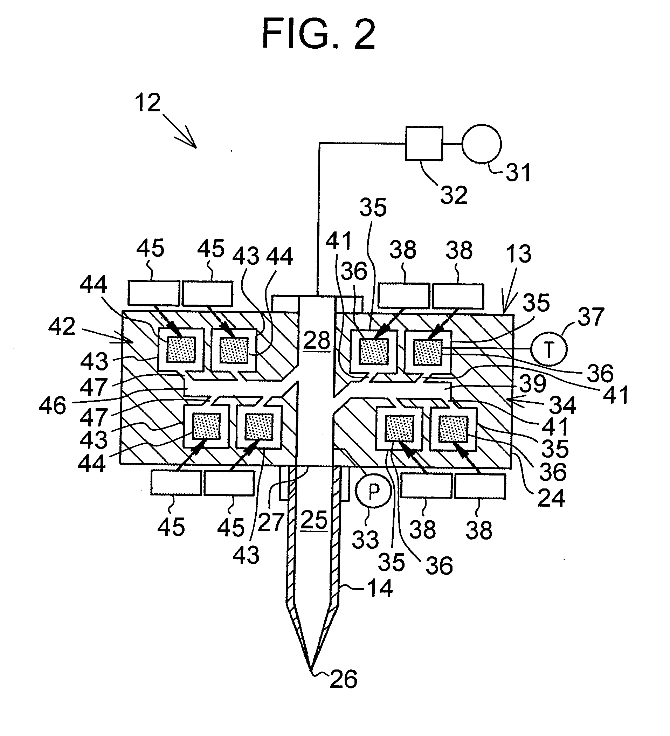

FIG. 2 shows the structure of the infusion unit 12. As shown in FIG. 2, the infusion unit 12 is provided with a supporting unit 24, the end of which supports the capillary 14. The capillary 14 is removably attached to the end of the supporting unit 24. A capillary conduit (or channel) 25 that defines a conduit of a liquid is formed in the capillary 14. The capillary conduit 25 extends along the axis of the capillary 14 from the proximal end, that is, an upstream end to the tip end, that is, a downstream end. The capillary 14 is tapered towards the tip end. An infusion port (or hole) 26 is formed at the tip of the capillary 14. The infusion port 26 has an inner diameter of the order of 0.8 µm. The proximal end of the capillary 14 is connected to an injection port 27 provided on the end of the supporting unit 24. The injection port 27 corresponds to the tip end of a first conduit (or channel) 28 formed in the supporting unit 24. In this way, the proximal end of the capillary conduit 25 is connected to the tip end of the first conduit 28. The first conduit 28 extends in the axial direction of the capillary 14.

A pressure pump 31 is connected to the proximal end of the first conduit 28. The pressure pump 31 supplies a predetermined pressure to the first conduit 28 and the capillary conduit 25. The pressure generated by the pressure pump 31 is kept at a predetermined level. A regulator 32 is provided between the first conduit 28 and the pressure pump 31. The pressure in the first conduit 28 and the capillary conduit 25 is adjusted with the aid of the regulator 32. A pressure sensor 33 is provided in the supporting unit 24. The pressure sensor 33 detects the pressure in the first conduit 28. The pressure sensor 33 may be, for example, an optical fiber pressure sensor. The optical fiber pressure sensor includes an optical fiber. A film is formed at the tip of the optical fiber. The film formed on the tip of the optical fiber deforms in accordance with the pressure in the first conduit 28. The deformation of the film causes a light interference phenomenon (or pattern). A target pressure is detected based on the interference phenomenon. In other words, the pressure may be measured based on the interference phenomen.

The supporting unit 24 including a positive pressure generating mechanism 34. The positive pressure generating mechanism 34 includes four first chambers 35, for example. Each first chamber 35 is filled with at least one rare gas (noble gas) such as argon or helium and nitrogen. Each first chamber 35 includes a first heat generator 36. A converting element for converting light energy to heat energy is used as the first heat generator 36. A temperature sensor 37 is connected to at least one of the first heat generators 36. The temperature sensor 37 detects the temperature of the first heat generators 36. A first irradiation mechanism 38 is provided opposite to each first heat generator 36. The first irradiation mechanism 38 applies, for example, pulsed laser light to the first heat generators 36. When pulsed laser light is applied to the first heat generators 36 they generate heat. Any black body that easily absorbs heat can be used as the converting element.

Each first chamber 35 is connected to a second conduit (or channel) 39 formed in the supporting unit 24. The second conduit 39 extends in a direction orthogonal (perpendicular) to the first conduit 28 and merges with the first conduit 28 at the tip end, that is, the downstream end. The second conduit 39 reduces a distance from the first conduit 28 toward the infusion port 26 of the capillary 14 and then merges with the first conduit 28. The second conduit 39 is open only at the tip end. The first chamber 35 and the second conduit 39 are connected together with a branch conduit (or channel) 41. The branch conduit 41 reduces a distance from the second conduit 39 toward the tip end of the second conduit 39 and merges with the second conduit 39. The first chamber 35 is open only at the branch conduit 41. The branch conduit 41 merges with the second conduit 39 at regular intervals from the proximal end, that is, the upstream end to the tip end.

Likewise, the supporting unit 24 includes a negative pressure generating mechanism 42. The negative pressure generating mechanism 42 includes four second chambers 43, for example. Each second chamber 43 is filled with at least one rare gas (noble gas) such as argon or helium and nitrogen. Each second chamber 43 includes a second heat generator 44. A converting element for converting light energy to heat energy is used as the second heat generator 44. A second irradiation mechanism 45 is provided opposite to each second heat generators 44. The second irradiation mechanism 45 applies, for example, pulsed laser light to the second heat generators 44. When pulsed laser light is applied to the second heat generators 44 they generate heat. Any black body that easily absorbs heat can be used as the converting element.

Each second chamber 43 is connected to a third conduit (or channel) 46 formed in the supporting unit 24. The third conduit 46 extends in a direction orthogonal (perpendicular) to the first conduit 28 and merges with the first conduit 28 at the tip end, that is, the downstream end. The third conduit 46 reduces a distance from the first conduit 28 toward a direction away from the infusion port 26 of the capillary 14 and then merges with the first conduit 28. The third conduit 46 may merge with the first conduit 28 in a position closer to the upstream side of the first conduit 28 than that of the second conduit 39. The second chamber 43 and the third conduit 46 are connected together with a branch conduit (or channel) 47. The branch conduit 47 reduces a distance from the third conduit 46 toward the tip end of the third conduit 46 and merges with the third conduit 46. The second chamber 43 is open only at the branch conduit 47. The branch conduit 47 merges with the third conduit 46 at regular intervals from the proximal end, that is, the upstream end to the tip end.

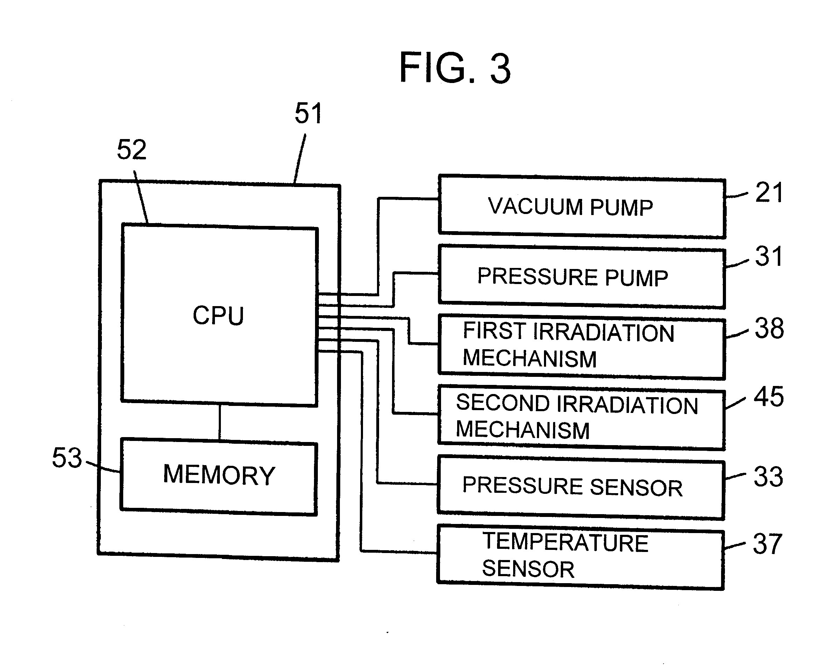

FIG. 3 is a block diagram of a control system of the microinjection apparatus 11 of this embodiment. As shown in FIG. 3, the microinjection apparatus 11 includes a computer 51. The computer 51 includes a CPU (central processing unit) 52, a memory 53, and other such electronic circuit elements. The CPU 52 executes various types of calculation based on software programs or data temporarily stored in the memory 53. Such software programs or data may be stored in a large-capacity storage device such as a hard disk drive (HDD) incorporated to the computer 51.

The CPU 52 outputs controls signal to control the driving of the vacuum pump 21 or the pressure pump 31. Likewise, the first irradiation mechanism 38 or the second irradiation mechanism 45 turns on/off pulse laser light, that is, starts/stops the application of the laser light based on control signals output from the CPU 52. On the other hand, pressure or temperature information is sent from the pressure sensor 33 or the temperature sensor 37 to the CPU 52. As described below, the CPU 52 controls the driving of the first irradiation mechanism 38 and the second irradiation mechanism 45 based on the pressure or temperature information. In this way, the CPU feedback-controls the infusion unit 12.

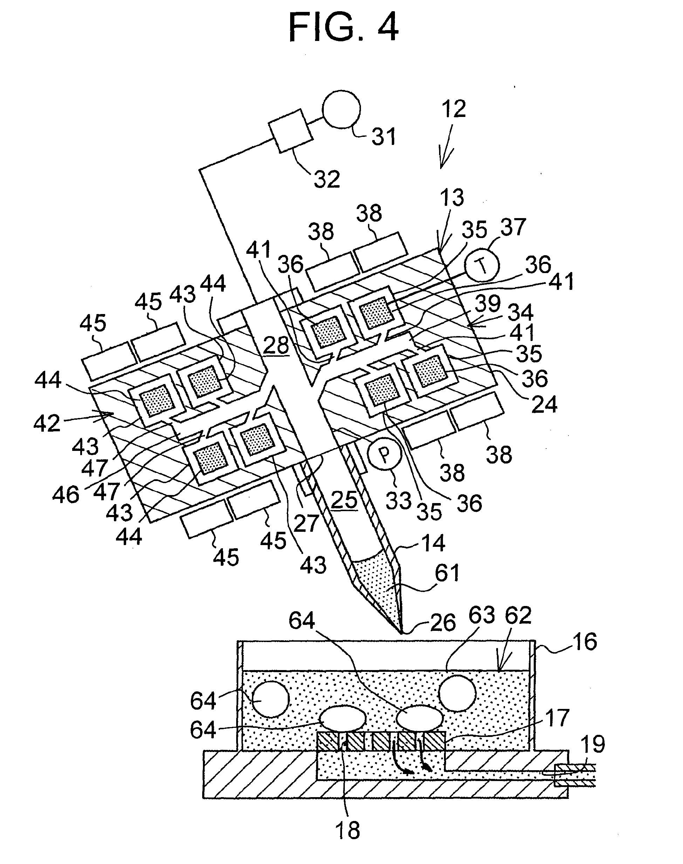

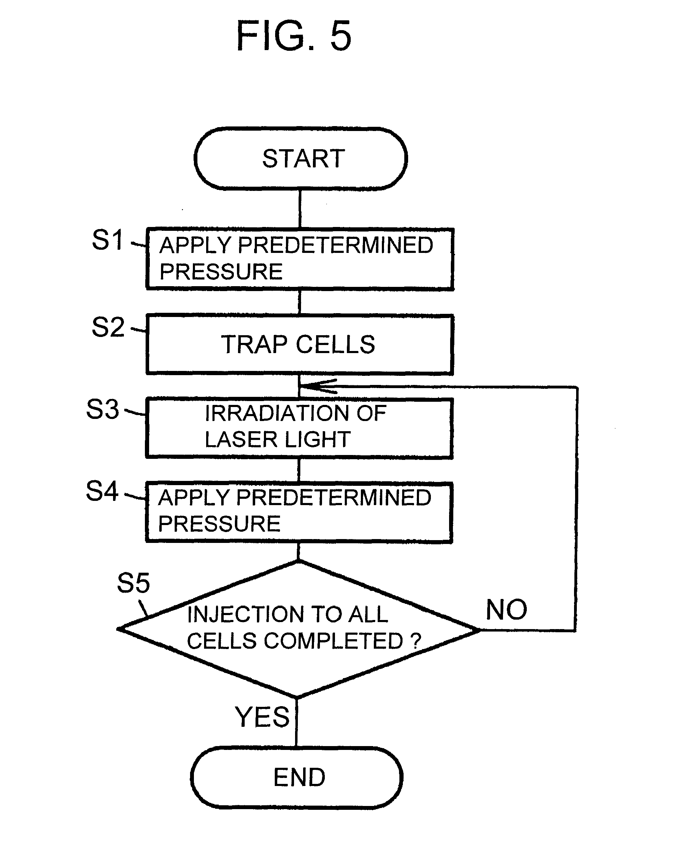

Consider that a medical agent is to be injected into a cell. The capillary 14 is attached to the injection port 27 of the supporting unit 24. As shown in FIG. 4, a predetermined amount of liquid, that is, medical agent 61 is previously held at the tip of the capillary 14. The interface of the medical agent 61 is defined on the downstream side of the proximal end of the capillary 14. In this way, the second conduit 39 and the third conduit 46 merge with the first conduit 28 on the upstream side of the interface of the medical agent 61. FIG. 5 is a flowchart of a procedure of injecting medical agent into a cell according to this embodiment. In step S1 of FIG. 5, the CPU 52 drives the pressure pump 31. As the pressure pump 31 is driven, a predetermined pressure is applied to the upstream end of the first conduit 28. Thus, a pressure in the capillary conduit 25, the first conduit 28, the second conduit 39, the third conduit 46, the first chamber 35, and the second chamber 43 is kept at a predetermined level. This pressure acts on the interface of the medical agent 61. The medical agent 61 is held on the tip of the capillary 14 due to the pressure. This pressure is set such that the medical agent 61 is prevented from being ejected from the infusion port 26 of the capillary 14.

On the other hand, the dish 16 is placed on the level surface of the operation stage 15. A drop of suspension 62 is put into the dish 16. The suspension 62 includes a culture solution 63 and cells 64 dispersed in the culture solution 63. The CPU 52 drives the vacuum pump 21 with the dish 16 being placed on the operation stage 15. As the vacuum pump 21 is driven, a negative pressure is generated in the path 19. The pressure regulating valve 22 keeps the negative pressure in the path 19 at a predetermined level. Due to the negative pressure generated in the path 19, the culture solution 63 is sucked out of the dish 16 towards the path 19 through the through-holes 18. Therefore the culture solution 63 flows into the tank 23. The culture solution 63 is stored in the tank 23 in order to prevent intrusion of the culture solution 63 into the pressure regulating valve 22 or the vacuum pump 21. In step S2, the cells 64 dispersed in the culture solution 63 are adsorbed at a predetermined position. In other words, cells are held over the through-holes 18 due to the negative pressure applied by the pump 21.

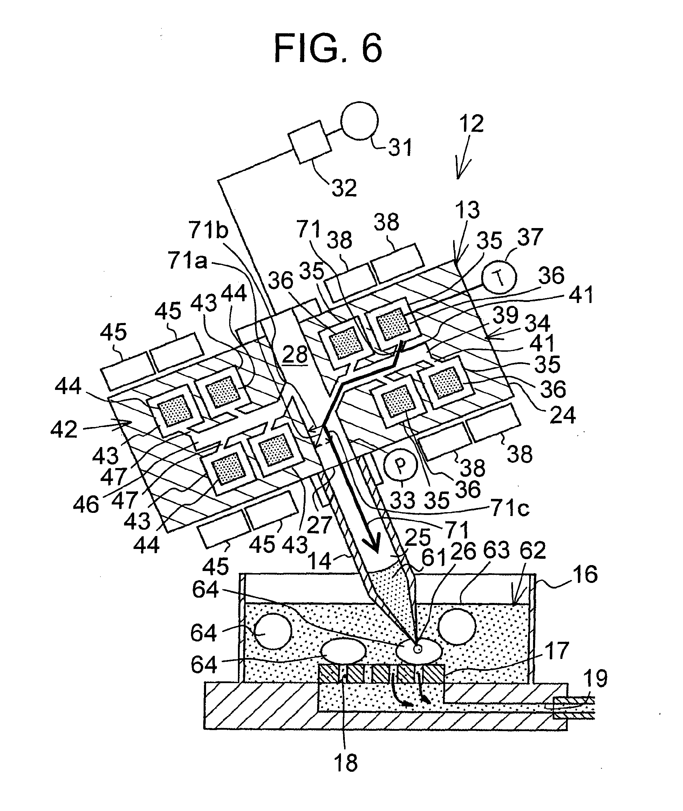

As the infusion unit 12 moves forward, the capillary 14 is inserted into the culture solution 63 in the dish 16. As shown in FIG. 6, the tip end of the capillary 14 is thereby inserted to the cell 64. In such a state, the CPU 52 drives the positive pressure generating mechanism 34. In step S3, the CPU 52 outputs a control signal to a first irradiation mechanism 38. In response to the control signal, the first irradiation mechanism 38 applies pulses of laser light to the first heat generator 36 under predetermined irradiation conditions. Due to the application of laser light, the first heat generator 36 generates heat. Along with the heat generation of the first heat generator 36, the temperature of a gas in the first chamber 35 increases. Along with the temperature rise, the gas expands. As a result, a pressure in the first chamber 35 increases. The first chamber 35 is open only at the branch conduit 41, so an airflow 71 is generated in a direction from the first chamber 35 to the second conduit 39 in accordance with a pressure difference between the first chamber 35, and the first conduit 28 and the second conduit 39. The branch conduit reduces a distance from the second conduit 39 toward the tip end of the second conduit 39 and then merges with the second conduit 39, so the airflow 71 moves towards the downstream end of the second conduit 39. Thus, the airflow 71 joins the first conduit 28.

The second conduit 39 reduces a distance from the first conduit 28 toward the infusion port 26 and then merges with the first conduit 28, so the airflow 71 is generated toward the injection port 27, that is, the infusion port 26 along an inner wall of the first conduit 28. The second conduit 39 extends toward the downstream end of the first conduit 28 with a certain angle to the first conduit 28. Thus, the airflow 71 generated in a direction from the second conduit 39 to the first conduit 28 is expressed by an inclination component 71a that crosses the axis of the first conduit 28 at a predetermined inclination angle. The inclination component 71a is expressed by an axial component 71c extending to the downstream end of the first conduit 28 in the axial direction of the first conduit 28 and an orthogonal component 71b extending in a direction orthogonal to the axis of the first conduit 28. The airflow 71 of the axial component 71c acts on the interface of the medical agent 61. A pressure acting on the medical agent 61 increases due to the airflow 71. The airflow 71 is turned into an injection pressure. As a result, a very small amount of medical agent 61 is injected from the infusion port 26 into the cell 64.

After the injection of the medical agent 61, the infusion unit 12 moves backward away from the culture solution 63. At this time, the pressure pump 31 applies a predetermined pressure to the first conduit 28 and the capillary conduit 25. After that, in step S5, the CPU 52 checks whether injection to all the cells 64 is completed. After the completion of injecting the agent to all the cells 64, the processing of the CPU 52 is terminated. On the other hand, if injection to any cell 64 is not completed, the CPU 52 repeats the processing from step S3.

At the time of setting the above pulse laser light irradiation conditions, the CPU 52 references the pressure information sent from the pressure sensor 33. At the same time, the CPU 52 references the temperature information sent from the temperature sensor 37. Based on the referenced information, the CPU 52 determines a relationship between a laser light irradiation time and its output value, temperature rise of the first heat generator 36, and a pressure of the first conduit 28. Such a relationship may be determined prior to the injection. The previously determined relationship may be stored in the memory 53. In this way, an injection amount of the medical agent 61 is determined based on how much a pressure of the first conduit 28 and a pressure of the capillary conduit 25 increase. Besides, the CPU 52 may reference pressure information and temperature information at the time of outputting control signals. Even if this causes an error due to any external factor such as a change in ambient temperature, a pressure increase can be precisely controlled through a so-called feedback control.

According to the thus-structured microinjection apparatus 11, heat generation of the first heat generator 36 is utilized for applying an injection pressure. The temperature of a gas in the first chamber 35 increases and the gas expands along with the heat generation of the first heat generator 36. In this way, a pressure in the first chamber 35 increases. As a result, the airflow 71 is generated in a direction from the first chamber 35 to the second conduit 39. The second conduit 39 reduces a distance from the first conduit 28 toward the infusion port 26 and then merges with the first conduit 28, so the airflow 71 from the first chamber 35 moves toward the infusion port 26. Such generation of the airflow 71 increases a pressure applied to the interface of the medical agent 61. As a result, an injection pressure is applied to the first conduit 28 and the capillary conduit 25 to eject very small of medical agent 61 from the infusion port 26. In this way, the airflow 71 is utilized for the application of the injection pressure, with the result that intrusion of a foreign material to the medical agent 61 can be securely prevented.

The inventors of the present invention have calculated an injection amount of medical agent by simulation. The calculation is performed under the following conditions. That is, an output power of the first irradiation mechanism 38 is set to 1 [W]. A pulse laser light irradiation time is set to 10 [ms] (1 ms x 10 pulses). Provided that an energy conversion efficiency of the first heat generator 36 is 100 [%], 10 [mj] of heat energy is applied to a gas in the first chamber 35. Nitrogen is used as the gas under such a condition that an isovolumetric specific heat rate Cv = 0.736 [J/gK] and density = 1.250 [g/l] (at 0°C and 1 [atm]). A nitrogen gas having a volume of 1 [ml] has a weight of 1.250 [mg]. At this time, the temperature of nitrogen in the first chamber 35 is raised from 0 [°C] to 10.9 [°C] based on the above heat energy. At this time, a pressure is changed almost in proportion to change in absolute temperature, so the pressure of nitrogen in the first chamber 35 is increased from 1.00 [atm] to 1.04 [atm]. In this way, a rapid pressure increase of 0.04 [atm] (≅ 4 kPa) is attained. In this way, if the medical agent 61 is water, water is injected in several tens of femtoliter from the capillary 14. In this way, it was confirmed that very small injection amount could be set.

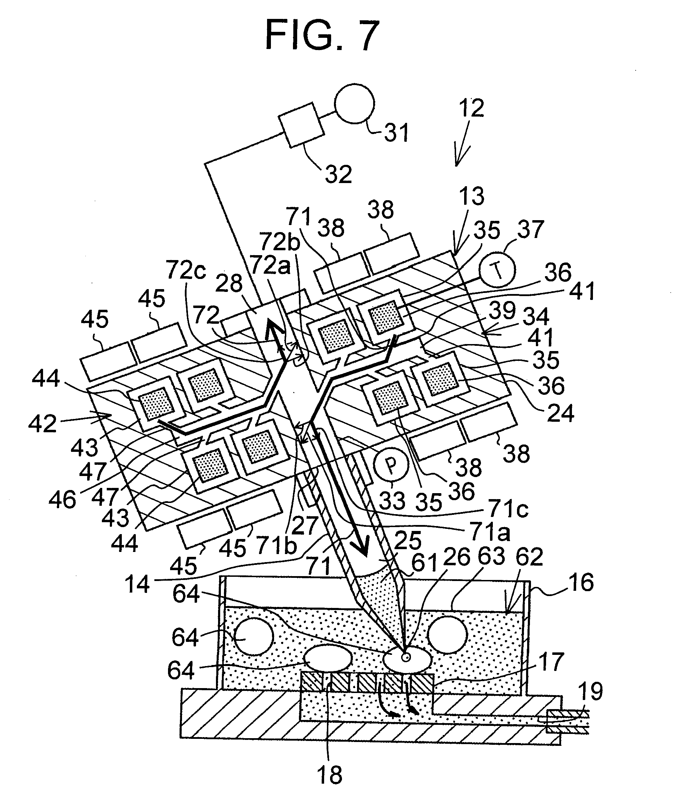

Upon adjusting an injection pressure, the negative pressure generating mechanism 42 may be driven. In this case, the negative pressure generating mechanism 42 may be driven together with the positive pressure generating mechanism 34. The CPU 52 outputs a control signal to the second irradiation mechanism 45 as well as the first irradiation mechanism 38. The second irradiation mechanism 45 applies pulse laser light to the second heat generator 44 under predetermined irradiation conditions. An output power of the pulse laser light is set lower than that of pulse laser light applied to the first heat generator 36. In this way, the second heat generator 44 generates heat. Along with the heat generation of the second heat generator 44, the temperature of a gas in the second chamber 43 increases. Along with the temperature rise, the gas expands. As a result, a pressure in the second chamber 43 increases. Similar to the first chamber 35, the second chamber 43 is open only at the branch conduit 47, so as shown in FIG. 7, an airflow 72 is generated in a direction from the second chamber 43 to the third conduit 46 in accordance with a pressure difference between the second chamber 43, and the first conduit 28 and the third conduit 46. The branch conduit 47 reduces a distance from the third conduit 46 toward the tip end of the third conduit 46 and merges with the third conduit 46, so the airflow 72 moves toward the tip end of the third conduit 46. In this way, the airflow 72 joins the first conduit 28.

The third conduit 46 reduces a distance from the first conduit 28 toward a direction away from the infusion port 26 and then merges with the first conduit 28, so the airflow 72 is generated toward the upstream end of the first conduit 28 along an inner wall of the first conduit 28. The third conduit 46 extends towards the upstream end of the first conduit 28 with a certain angle to the first conduit 28, so the airflow 72 generated in a direction from the third conduit 46 to the first conduit 28 is expressed by an inclination component 72a that crosses the axis of the first conduit 28 at a predetermined inclination angle. The inclination component 72a is expressed by an axial component 72c extending to the upstream end of the first conduit 28 in the axial direction of the first conduit 28 and an orthogonal component 72b extending in a direction orthogonal to the axis of the first conduit 28. The airflow 72 of the axial component 72c moves in a direction opposite to the medical agent 61. The airflow 71 reduces a pressure generated with the positive pressure generating mechanism 34 and acting on the interface of the medical agent 61. In other words, a negative pressure generated with the negative pressure generating mechanism 42 is applied to the interface of the medical agent 61. An output power of that of pulse laser light applied to the second heat generator 44 is set smaller than that of pulse laser light applied to the first heat generator 36, so the negative pressure generated with the negative pressure generating mechanism 42 and acting on the interface of the medical agent 61 due to the airflow 72 and the positive pressure acting on the interface of the medical agent 61 due to the airflow 71 could cancel each other. As a result, a positive pressure corresponding to a difference between the positive pressure and the negative pressure acts on the interface of the medical agent 61, and the medical agent 61 is injected from the infusion port 26. In this way, an injection pressure acting on the interface of the medical agent 61 is reduced compared with the case of using the positive pressure generating mechanism 34 alone. As a result, a smaller amount of medical agent 61 is injected from the infusion port 26. An injection pressure can be finely adjusted by controlling the positive pressure and the negative pressure in this way.

On the other hand, the negative pressure generating mechanism 42 may be driven at the time of stopping the injection of the medical agent 61 to the cells 64. In this way, at the time of injecting the medical agent 61, the CPU 52 outputs a control signal to the second irradiation mechanism 45. The second irradiation mechanism 45 irradiates the second irradiation mechanism 45 with pulse laser light under predetermined irradiation conditions. An output power of the pulse laser light is set equal to that of the pulse laser light applied to the first heat generator 36. The second heat generator 44 generates heat. As in the above example, the airflow 72 is generated in a direction from the second chamber 43 to the third conduit 46. The airflow 72 moves towards the upstream end of the first conduit 28 along an inner wall of the first conduit 28. As a result, a negative pressure acts on the interface of the medical agent 61. An output power of the pulse laser light applied to the second heat generator 44 is set equal to that of the pulse laser light applied to the first heat generator 36, so the negative pressure acting on the interface of the medical agent 61 due to the airflow 72 is equal to the positive pressure acting on the interface of the medical agent 61 due to the airflow 71. The positive pressure and the negative pressure completely cancel each other. Thus, the injection pressure applied to the interface of the medical agent 61 is set to zero. As a result, the injection of the medical agent 61 is stopped. The injection of the medical agent 61 to the cells 64 can be stopped by adjusting the positive pressure and the negative pressure in this way.

Further, pulse laser light may be sequentially applied to the plural first heat generators 36 upon the adjustment of an injection pressure. If a single first heat generator 36 is continuously applied with pulse laser light, the generator is heated all this while. As a result, the first heat generator 36 takes a long time to dissipate the heat. If the first heat generator 36 cannot dissipate the heat well, a large temperature difference of the first heat generator 36 cannot be obtained between before and after pulse laser light application. Thus, in order to precisely control an injection amount of the medical agent 61, the first heat generator 36 needs to dissipate the heat enough. However, since the first heat generator 36 takes a long time to dissipate the heat, there is a possibility of lowering efficiency of injection of the medical agent 61 to the cells 64. According to the embodiment, such a problem can be solved.

First, pulse laser light is applied to the first heat generator 36 upon injection of the medical agent 61 to the cell 64. After the completion of the injection, the second first heat generator 36 is irradiated with pulse laser light for injection of the medical agent 61 to the next cell 64. From then on, the third one, the fourth one, the first one, ..., of the first heat generators 36 are irradiated with pulse laser light upon each injection. As a result, it is possible to prevent such a situation that the temperature of any one of the first heat generators 36 increases excessively. Each first heat generator 36 has enough time to dissipate heat. A temperature rise of a gas can be precisely controlled with the thus-controlled first heat generators 36. Thus, an injection amount of the medical agent 61 is precisely controlled. At the time of adjusting the injection pressure, pulse laser light may be successively applied to the plural second heat generators 44 to generate a negative pressure.

On the other hand, pulse laser light may be applied to the plural first heat generators 36 at the same time upon adjusting an injection pressure. For example, if pulse laser light is applied to the four first heat generators 36 at the same time, gases in the first chambers 35 expand at the same time. As a result, as compared with the case of applying pulse laser light to only one first heat generator 36, an airflow is generated at a high flow rate. Then, a higher pressure acts on the interface of the medical agent 61. In this way, a high injection pressure is applied to the first conduit 28 and the capillary conduit 25. As a result, an injection amount of the medical agent 61 can be increased. Moreover, even though a high power of pulse laser light is not applied to one first heat generator 36 for a long time, a high injection pressure can be obtained. As a result, it is possible to prevent such a situation that the temperature of any one of the first heat generators 36 increases excessively. Similar to the above example, at the time of adjusting the injection pressure, pulse laser light may be successively applied to the plural second heat generators 44 to generate a negative pressure.

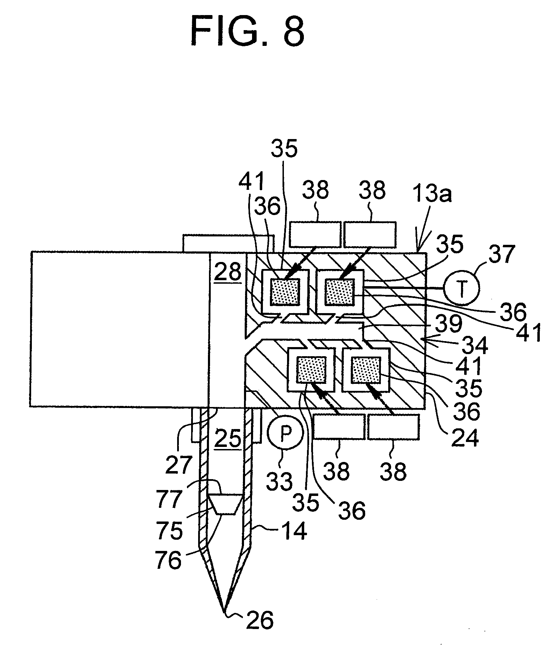

As shown in FIG. 8, the microinjection apparatus 11 may incorporate a liquid injection apparatus 13a in place of the liquid discharge apparatus 13. In the liquid discharge apparatus 13a of FIG. 8, an upstream end of the first conduit 28 is closed. In other words, the pressure pump 31 is omitted. In the supporting unit 24, the negative pressure generating mechanism 42 is omitted. In the case of applying a predetermined pressure or an injection pressure to the first conduit 28 and the capillary conduit 25, the branch conduit 41 generates a positive pressure. However, as the first heat generators 36 are cooled after heat radiation, a negative pressure is generated in the first conduit 28 or the capillary conduit 25. The generation of the negative pressure causes inflow of the culture solution 63 from the infusion port 26 or back-flow of the medical agent 61 in the capillary 14. Such a phenomenon hinders injection of the medical agent 61. To prevent such generation of the negative pressure, for example, a check valve 75 is provided to the capillary 14. Identical or equivalent components or structures to those of the liquid discharge apparatus 13 are denoted by like reference numerals.

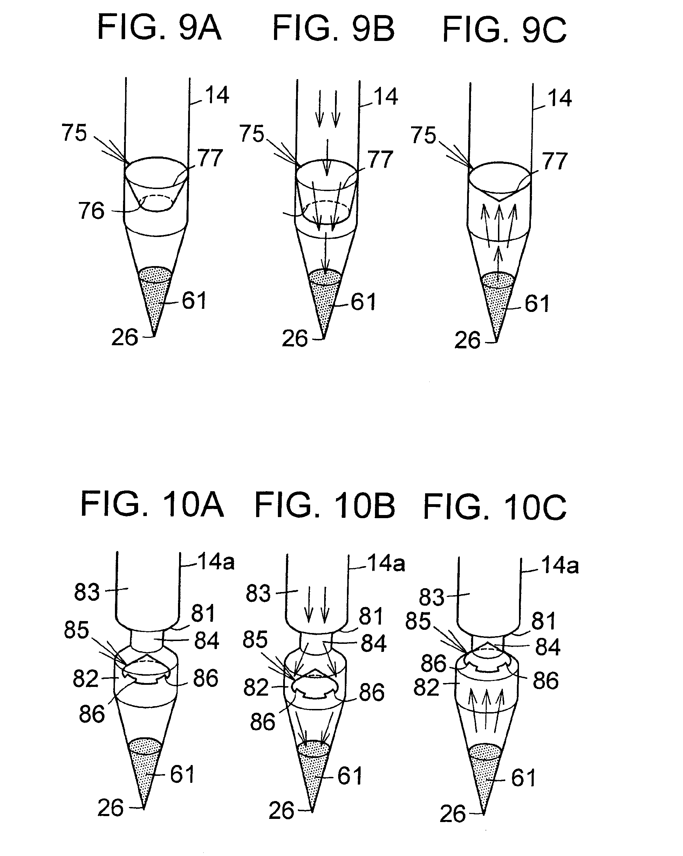

FIGs. 9A to 9C show the structure of the check valve 75. As shown in FIG. 9A, the check valve 75 has a truncated cone shape. The check valve 75 has a diameter that increases from the downstream end of the capillary 14 towards the upstream end. In this way, the check valve 75 defines a leading-end opening 76 having a first diameter and a proximal-end opening 77 having a second diameter larger than the first diameter. The check valve 75 is attached to the inner peripheral surface of the capillary 14 at (or towards) the proximal end. The check value 75 is made of an elastic resin material such as an elastomer resin. If an injection pressure is applied to the first conduit 28 and the capillary conduit 25, the leading-end opening 76 of the check valve 75 flares widely (i.e. the diameter of opening 76 increases) along with elastic deformation thereof as shown in FIG. 9B. Thus, the injection pressure acts on the interface of the medical agent 61. On the other hand, if a negative pressure is applied to the first conduit 28 and the capillary conduit 25, as shown in FIG. 9C, the leading-end opening 76 of the check valve 75 is closed (i.e. the diameter of opening 76 decreases) along with elastic deformation thereof. As a result, the inflow of the culture solution 63 or the back-flow of the medical agent 61 can be avoided. The check valve 75 may be provided in the first conduit 28. At this time, the check valve 75 may be provided on the downstream side of a position where the first conduit 28 merges with the second conduit 39.

FIGs. 10A to 10C show another example of the capillary 14 and the check valve 75. As shown in FIG. 10A, the infusion unit 12 incorporates a capillary 14a in place of the capillary 14. A constriction 81 is formed around the capillary 14a in the axial direction. As a result, large-diameter portions 82 and 83 having a first diameter and a small-diameter portion 84 having a second diameter smaller than the first diameter are formed in the capillary 14a. A check valve 85 is provided in the large-diameter portion 82. The check valve 85 has a conical shape, for example. The maximum diameter of the check valve 85 may be set smaller than the first diameter and larger than the second diameter. Plural cutouts 86 are formed around the check valve 85, for example. The diameter of the inner edge of each cutout 86 is set larger than the second diameter. As in the above case, the check valve 85 is made of an elastic resin material such as an elastomer resin. The check valve 85 may be provided in the first conduit 28. At this time, the check valve 85 may be provided on the downstream side of a position where the first conduit 28 and the second conduit 39 merge with each other.

In the capillary 14a, if an injection pressure is applied to the first conduit 28 and the capillary conduit 25, as shown in FIG. 10B, the check valve 85 moves toward the downstream end of the capillary 14a. As a result, an injection pressure is applied from the large-diameter portion 83 to the large-diameter portion 82 through the small-diameter portion 82. In this way, the injection pressure acts on the interface of the medical agent 61. Since the cutouts 86 are formed in the check valve 85, even if the check valve 85 is pressed against the tip end of the capillary 14a, an injection pressure securely acts on the downstream end of the capillary 14a from the cutouts 86. On the other hand, if a negative pressure is applied to the first conduit 28 and the capillary conduit 25, the check valve 85 has a maximum diameter larger than the second diameter of the second-diameter portion 84. Thus, the check valve 85 is pressed against the small-diameter portion 84. As a result, as shown in FIG. 10C, the inflow of the culture solution 63 and the backflow of the medical agent 61 can be prevented.

In addition, as the heat generator, a heater such as heating wire or another type of heat generator that combines a magnetic coil and metal may be used. As is well known, if a current flows through a heating wire, the current is converted into heat in accordance with the resistance of the heating wire. In this way, the heating wire generates heat. On the other hand, the combination of a magnetic coil and metal realizes electromagnetic-induction heating. As is well known, if a current flows through a magnetic coil, a magnetic field is generated from the magnetic coil. The generated magnetic field acts on a metal. As a result, an eddy current is generated in the metal. The eddy current flowing through the metal is converted to heat in accordance with the resistance of the metal. In this way, the metal generates heat.

A discharge apparatus according to an embodiment includes: a discharge outlet for discharging the liquid; a first conduit extending toward the discharge outlet; a second conduit having one end connected to the first conduit for flowing a gas therethrough, the second conduit extending generally in the direction toward the discharge outlet and generally approaching the first conduit toward the portion where the second conduit is connected to the first conduit; a chamber connected to the other end of the second conduit; and a heater installed to the chamber for heating a gas in the chamber so as to cause the heated gas to flow into the second conduit and to push out the liquid out of the discharge outlet.

In the liquid injection apparatus, a plurality of chambers may be connected to the second conduit. In such a liquid injection apparatus, heat generators generate heat in order in the plurality of chambers. Along with heat generation, airflows are successively generated. In this way, an injection pressure is intermittently applied to a capillary, for example. For example, a liquid is successively injected to a plurality of objects of liquid injection. The plurality of heat generators are used as above, so it is possible to prevent an excessive temperature rise in one heat generator and secure enough time to dissipate heat of the heat generator. On the other hand, heat generators may generate heat in a plurality of chambers at the same time. In this case, the degree of temperature increase is higher than that of one heat generator. As a result, a large airflow is generated. A high injection pressure is applied to the capillary.

The discharge apparatus may further include a check valve provided in the first conduit and controls a back-flow of a liquid from the injection port to the first conduit. In the liquid injection apparatus, a negative pressure is generated in the first conduit as the heat generator is cooled after heat radiation. Owing to the provision of the check valve, the back-flow of a liquid to the first conduit from the injection port is prevented even if a negative pressure is generated in the first conduit. For example, the back-flow of the liquid to the capillary is prevented.

The discharge apparatus may further include: a third conduit having one end connected to the first conduit for flowing a gas therethrough, the third conduit extending generally in the direction toward the discharge outlet and generally approaching the first conduit toward the portion where the second conduit is connected to the first conduit;
another chamber connected to the other end of the third conduit; and a heater the another chamber so as to cause the heated gas to flow into the third conduit and to control to push out the liquid out of the discharge outlet.

In this case, a plurality of auxiliary chambers may be connected to the third conduit. As in the above case, heat generators generate heat in order in the plurality of auxiliary chambers. Along with heat generation, airflows are successively generated. In this way, a negative pressure is intermittently applied to the injection port, for example. Such a negative pressure can be used for controlling the injection pressure. The plurality of heat generators are used as above, so it is possible to prevent an excessive temperature rise in one heat generator and secure enough time to dissipate heat of the heat generator. On the other hand, heat generators may generate heat in a plurality of chambers at the same time. In this case, the degree of temperature increase is higher than that of one heat generator. As a result, a large airflow is generated. A high injection pressure is applied to the capillary.

The thus-structured discharge apparatus is incorporated in, for example, an injection apparatus. The injection apparatus includes: a capillary having one end for injecting the liquid; a first conduit extending toward the other end of the capillary; a second conduit having one end connected to the first conduit for flowing a gas therethrough, the second conduit extending generally in the direction toward the discharge outlet and generally approaching the first conduit toward the portion where the second conduit is connected to the first conduit; a chamber connected to the other end of the second conduit; and a heater installed to the chamber for heating a gas in the chamber so as to cause the heated gas to flow into the second conduit and to push out the liquid out of the capillary. According to the injection apparatus, the same advantages as above can be attained.

An injection method according to another embodiment includes: generating heat with a heat generator installed to a chamber containing a gas so as to cause expansion of the gas, the chamber being connected to a second conduit connected to a first conduit connected to a capillary having an end for discharging the liquid therefrom; and generating an airflow from the second conduit toward another end of the capillary along with the expansion of the gas so as to increase a pressure acting on the liquid to discharge the liquid from the end of the capillary. Another chamber may connect to the second conduit and a heat generator may installed in the another chamber.

Along with the generation of the airflow, heat generators generate heat in order in a plurality of chambers. Along with heat generation, airflows are successively generated. In this way, an injection pressure is intermittently applied to the tip end of a capillary, for example. For example, a liquid is successively injected to a plurality of objects of liquid injection. The plurality of heat generators are used as above, so it is possible to prevent an excessive temperature rise in one heat generator and secure enough time to dissipate heat of the heat generator. On the other hand, heat generators may generate heat in a plurality of chambers connected to the second conduit at the same time upon the generation of the airflow. In this case, the degree of temperature increase is higher than that of one heat generator. As a result, a large airflow is generated. A high injection pressure is applied to the capillary.

The injection method may further include: generating heat with a heat generator in an auxiliary chamber so as to cause expansion of a gas in the chamber, the auxiliary chamber being connected to a third conduit connected to the first conduit; and generating an airflow from the third conduit to the first conduit, the airflow being in a direction opposite to the end of the capillary along with the expansion of the gas so as to reduce a pressure acting on the liquid to control injection of the liquid from the end of the capillary.

As in the above case, upon the generation of the airflow, heat generators may generate heat in order in a plurality of auxiliary chambers connected to the third conduit. As in the above case, along with heat generation, airflows are successively generated. In this way, a negative pressure is intermittently applied to the injection port, for example. Such a negative pressure can be used for controlling the injection pressure. The plurality of heat generators are used as above, so it is possible to prevent an excessive temperature rise in one heat generator and secure enough time to dissipate heat of the heat generator. On the other hand, upon the generation of the airflow, heat generators may generate heat in a plurality of auxiliary chambers connected to the third conduit at the same time. In this case, the degree of temperature increase is higher than that of one heat generator. As a result, a large airflow is generated. A high injection pressure is applied to the capillary.

An injection apparatus according to another embodiment, for injecting an introduction material into a minute object includes: a capillary having an injection port for being inserted to the minute object so as to inject the introduction material into the minute object, the injection port being provided at an end of the capillary; a first conduit extending toward the other end of the capillary; a chamber connected to the first conduit; a light source for applying a light beam; a heat generator installed to the chamber for heating a gas in the chamber through the application with the light beam so as to cause the heated gas to flow into the first conduit and to push out the introduction material out of the capillary; and a control unit for performing on/off control of the light source.

An injection apparatus according to another embodiment, for injecting an introduction material into a minute object includes: a capillary having an injection port for being inserted to the minute object so as to inject the introduction material into the minute object, the injection port being provided at an end of the capillary; a conduit connected to the injection port; a positive pressure generating unit connected to the conduit, for generating a positive pressure to be applied from the conduit toward the injection port, the positive pressure generating unit having a first chamber of a gas, and a first heat generator for heating the first chamber; a negative pressure generating unit connected to the conduit, for generating a negative pressure to be applied from the conduit toward a direction away from the injection port, the negative pressure generating unit including a second chamber of a gas, and a second heat generator for heating the second chamber; and a control unit for controlling heat generation of the first heat generator and that of the second heat generator.

According to the embodiments, it is possible to provide a discharge apparatus, an injection apparatus and an injection method, which can inject very small amount of introduction material. In addition, any foreign material is prevented from mixing in the introduction material.

## Claims

1. A discharge apparatus for discharging a liquid comprising:
a discharge outlet for discharging the liquid;
a first conduit extending toward the discharge outlet;
a second conduit having one end connected to the first conduit for flowing a gas therethrough, the second conduit extending generally in the direction toward the discharge outlet and generally approaching the first conduit toward the portion where the second conduit is connected to the first conduit;
a chamber connected to the other end of the second conduit; and
a heater installed in the chamber for heating a gas in the chamber so as to cause the heated gas to flow into the second conduit and to push the liquid out of the discharge outlet.

2. The discharge apparatus according to claim 1, further comprising:
a further chamber connected to the second conduit.

3. The discharge apparatus according to claim 1 or 2, further comprising:
a check valve provided in the first conduit so as to regulate a back-flow of the liquid from the discharge outlet to the first conduit.

4. The discharge apparatus according to any preceding claim, further comprising:
a third conduit having one end connected to the first conduit for flowing a gas therethrough, the third conduit extending generally in the direction toward the discharge outlet and generally approaching the first conduit toward the portion where the second conduit is connected to the first conduit;
a chamber connected to the other end of the third conduit; and
a heater installed in the chamber so as to cause the heated gas to flow into the third conduit and to control the push of the liquid out of the discharge outlet.

5. The discharge apparatus according to claim 4, further comprising:
a further chamber connected to the third conduit.

6. The discharge apparatus according to any preceding claim, further comprising:
an open-ended pipe having one end as the discharge outlet and the other end connected to the first conduit; and
a supporting unit for supporting the open-ended pipe, the first conduit and the second conduit being formed within the supporting unit.

7. The discharge apparatus according to any of claims 4-6, further comprising:
an open-ended pipe having one end as the discharge outlet and the other end connected to the first conduit; and
a supporting unit for supporting the open-ended pipe, the third conduit being formed within the supporting unit.

8. The discharge apparatus according to any of claims 1-5, further comprising:
a capillary having one end as the discharge outlet for injecting the liquid.

9. The discharge apparatus according to claim 8, further comprising:
a supporting unit for supporting the capillary, the first conduit and the second conduit being formed within the supporting unit.

10. The discharge apparatus according to claim 8 or 9, further comprising:
a supporting unit for supporting the capillary, the third conduit being formed within the supporting unit.

11. The discharge apparatus according to any of claims 8-10, wherein the capillary is tapered toward the one end.

12. The discharge apparatus according to any preceding claim, further comprising:
a light source for applying a light beam;
a heat generator installed in the chamber for heating a gas in the chamber through the application of the light beam so as to cause the heated gas to flow into the first conduit and to push the liquid out of the discharge outlet; and
a control unit for performing on/off control of the light source.

13. An injection apparatus for injecting an introduction material into a minute object comprising:
a capillary having an injection port for being inserted to a minute object so as to inject an introduction material into the minute object, the injection port being provided at an end of the capillary;
a conduit connected to the injection port;
a positive pressure generating unit connected to the conduit, for generating a positive pressure to be applied from the conduit towards the injection port, the positive pressure generating unit having a first chamber of a gas, and a first heat generator for heating the first chamber;
a negative pressure generating unit connected to the conduit, for generating a negative pressure to be applied from the conduit toward a direction away from the injection port, the negative pressure generating unit including a second chamber of a gas, and a second heat generator for heating the second chamber; and
a control unit for controlling heat generation of the first heat generator and that of the second heat generator.

14. A method for discharging a liquid comprising:
generating heat with a heat generator installed in a chamber containing a gas so as to cause expansion of the gas, the chamber being connected to a second conduit connected to a first conduit connected to a capillary having an end for discharging the liquid therefrom; and
generating an airflow from the second conduit toward another end of the capillary along with the expansion of the gas so as to increase a pressure acting on the liquid to discharge the liquid from the end of the capillary.
